# EUROPEAN PATENT APPLICATION

(11) **EP 2 959 888 A1**
(43) Date of publication of application: **30.12.2015**
(21) Application number: 15173110.6
(22) Date of filing: 22.06.2015
(51) Int. Cl.: A61K 9/20, A61K 31/439, A61K 31/7056, A61P 31/14

(54) **A NOVEL PHARMACEUTICAL COMPOSITION OF SOFOSBUVIR AND RIBAVIRIN**

(30) Priority: 23.06.2014 TR 201407272; 24.06.2014 TR 201407287; 30.06.2014 TR 201407592
(71) Applicant: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); ERDEM, Yelda, 34460 Istanbul (TR); UCAR, Ezgi, 34460 Istanbul (TR); GÖKCEK, Sevgi, 34460 Istanbul (TR)
(74) Representative: Sevinç, Erkan

(57) **Abstract**

This invention is a novel pharmaceutical composition comprising sofosbuvir and ribavirin as active agents with at least one pharmaceutically acceptable excipient, wherein at least one of the active agents is in the form of controlled release, for use in the treatment of hepatitis C virus infections, chronic hepatitis C, hepatocellular carcinoma or patients with end-stage liver disease awaiting liver transplantation.

## Description

### Field of Invention

This invention is a novel pharmaceutical composition comprising sofosbuvir and ribavirin as active agents with at least one pharmaceutically acceptable excipient, wherein at least one of the active agents is in the form of controlled release.

More specifically, this invention relates to a novel pharmaceutical composition comprising sofosbuvir and ribavirin as active agents with at least one pharmaceutically acceptable excipient, wherein at least one of the active agents is in the form of controlled release, for use in the treatment of hepatitis C virus infections, chronic hepatitis C, hepatocellular carcinoma or patients with end-stage liver disease awaiting liver transplantation.

### Background of Invention

Chronic infection with hepatitis C virus (HCV) affects more than 170 million people worldwide and is a leading cause of anticipated liver-related death due to the development of cirrhosis and its complications. Hepatitis C virus (HCV) infection is a major health problem that leads to chronic liver disease, such as cirrhosis and hepatocellular carcinoma, in a substantial number of infected individuals, estimated by the World Health Organization to be about 3% of the world's population. An estimated 150-180 million individuals are chronically infected with HCV worldwide, with 3 to 4 million people infected each year. Once infected, about 20% of people clear the virus, but the rest can harbor HCV for the rest of their lives. Ten to twenty percent of chronically infected individuals eventually develop liver-destroying cirrhosis or cancer.

Presently there are numerous antiviral drugs which are widely available. In the last 10 years, standard of care anti-HCV treatment has been founded on the combination of Peginterferon (Peg-IFN) plus ribavirin (RBV), whose main disadvantages were suboptimal rates of sustained virological response (SVR) in difficult-to-treat patients (HCV genotype 1-4, advanced liver fibrosis) and, most of all, side effects profile resulting in poor tolerability and treatment contraindication in some patient subsets (decompensated liver disease and autoimmune disorders).2 The recent availability of culture cell models provided deeper insight in understanding HCV life cycle and was the basis for the development of new drugs targeting non-structural HCV proteins involved in viral replication process, such as nonstructural protein3 (NS3) and nonstructural protein 5A/B (NS5A/B). Direct-acting antivirals (DAAs) promised to open a new era in treating chronic HCV infection by increasing SVR rates, providing shortened and simplified regimens while also minimizing treatment-related side effects.

Sofosbuvir is a hepatitis C virus (HCV) nucleotide analog NS5B polymerase inhibitor indicated for the treatment of chronic hepatitis C (CHC) infection as a component of a combination. Sofosbuvir efficacy has been established in subjects with HCV genotype 1, 2, 3 or 4 infection, including those with hepatocellular carcinoma meeting Milan criteria (awaiting liver transplantation) and those with HCV/HIV-1 co-infection.

SOVALDI® is the brand name for sofosbuvir, a nucleotide analog inhibitor of HCV NS5B polymerase. The IUPAC name for sofosbuvir is (S)-Isopropyl 2-((S)-(((2R,3R,4R,5R)-5-(2,4-dioxo3,4-dihydropyrimidin-1 (2H)-yl)-4-fluoro-3-hydroxy-4-methyltetrahydrofuran-2yl)methoxy)-(phenoxy)phosphorylamino)propanoate. It has a molecular formula of C22H29FN3O9P and a molecular weight of 529.45. It has the following structural Formula I given below:

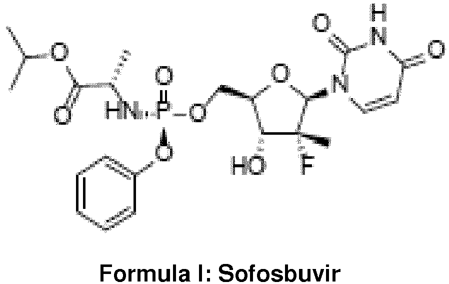

Sofosbuvir is a white to off-white crystalline solid with a solubility of ≥ 2 mg/mL across the pH range of 2-7.7 at 37°C and is slightly soluble in water. It is not metabolized by Cytochrome P450 (CYP450).

In prior art, EP2203462 (B1) discloses the compound of sofosbuvir. EP2432792 (A1) discloses a process for preparing nucleoside phosphoramidates and their use as agents for treating viral diseases. EP2552933 (A1) discloses crystalline or crystal-like form of sofosbuvir.

Ribavirin is a nucleoside analogue (purine analogue) with antiviral activity. COPEGUS® and REBETOL® are the brand names for ribavirin, The chemical name of ribavirin is 1-β-Dribofuranosyl-1 H-1,2,4-triazole-3-carboxamide and has the following structural Formula II given below:

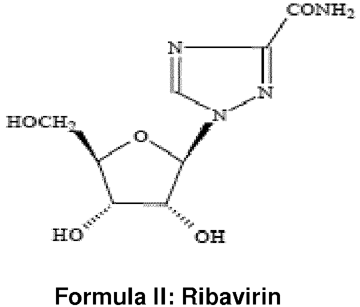

The empirical formula of ribavirin is C8H12N4O5 and the molecular weight is 242.21. Ribavirin is a white to off-white, crystalline powder. It is freely soluble in water and slightly soluble in anhydrous alcohol.

In prior art, US3798209 (A) discloses the compound of ribavirin. EP0093401 (B1) discloses a process for preparing ribavirin. EP0643970 (B1) discloses use of ribavirin in the medical treatment of viral diseases in humans.

However, clinical trials have shown that ribavirin alone can normalize W201 alanine aminotransferase (ALT) levels transiently during the course of treatment in some patients with chronic hepatitis C (CHC) infections. These studies have reported that ribavirin alone did not reduce HCV RNA levels during or after therapy and did not produce any sustained virologic response.

It is well known that drugs used in the same therapeutic area or even for treating the same indication cannot always be combined a priori with the expectation of at least additive therapeutic effects. The scientific literature is full of examples wherein compounds of different classes, which are used to treat the same indications, cannot be combined into safe and efficacious dosage forms thereby resulting in incompatible drug combinations. No pharmaceutical composition has been produced until today, which contains a combination of sofosbuvir and ribavirin. Even if some medicaments comprising either of these active agents have been administered concomitantly in practice, this fact requires the patients to carry more than one drug and causes application-related difficulties. Additionally, administering and formulating a combination, in place of the individual use of each active agent, may provide improved treatment features. Especially, controlled release compositions represent an alternative for such patients and provide for a better patient compliance.

Controlled-release dosage forms are designed to release a drug at a predetermined rate by maintaining a constant drug level for a specific period of time with minimum side effects. Compared to immediate release formulations, a controlled release formulation containing a physiologically active drug allows blood concentrations of the drug to be maintained for a long time or above the therapeutic concentration. By providing a controlled-release formulation of sofosbuvir and ribavirin, it may be possible to reduce the frequency of administration, while providing the same or better therapeutic effects, potentially improving compliance. The controlled-release formulation may avoid a rapid increase in blood plasma concentration levels immediately after administration of the drug, thus potentially reducing or eliminating adverse side effects.

Various formulations and methods are already known for the preparation of oral formulations of sofosbuvir or ribavirin. However, no controlled release pharmaceutical composition has been produced until today, which contains a combination of sofosbuvir and ribavirin. On the other hand, controlled release formulations are becoming an increasingly important issue in the area of better patient compliance comparative to the conventional solid dosage forms for oral administration such as capsules and tablets, which are the most commonly used. In particular, pediatric and geriatric patients and patients with mental problems. Thus, controlled release compositions represent an alternative for such patients and provide for a better patient compliance with long-term pharmaceutical therapies such as the treatment of hepatitis.

Thus, more need rises for oral controlled release formulations of sofosbuvir and ribavirin and a process for preparing such formulation which overcomes the above described problems in prior art and having additive advantages over them. According to these embodiments, there is provided a controlled-release formulation comprising sofosbuvir and ribavirin and at least one pharmaceutically acceptable excipient.

As a result, based on said drawbacks, a novelty is required in the art of pharmaceutical compositions having therapeutic effects against hepatitis C virus infections.

### Detailed description of the invention

The present invention provides a pharmaceutical composition comprising sofosbuvir and ribavirin as active agents with at least one pharmaceutically acceptable excipient, wherein at least one of the active agents is in the form of controlled release. This composition with synergistic action of sofosbuvir and ribavirin eliminating all before said problems and bringing additional advantages to the relevant prior art.
Another object of the present invention is to provide a pharmaceutical formulation making the plasma concentration level stable by maintaining release of sofosbuvir and ribavirin in the blood stream for a longer time period sufficient to justify once daily or twice daily dosing and thus increases patient compliance.
Another object of the present invention is to provide a pharmaceutical formulation of sofosbuvir and ribavirin comprising immediate release phase and controlled release phase comprising a rate controlling agent, for the treatment of hepatitis C virus infections by eliminating all the aforementioned drawbacks and providing additional advantages to the respective technical field.

Another object of the present invention is to obtain a composition with synergistic effect for use in the treatment of viral infections and preferably hepatitis C virus infections.

That provides once daily dosing for effective management of hepatitis C virus infections, chronic hepatitis C (CHC), hepatocellular carcinoma or patients with end-stage liver disease awaiting liver transplantation treating activity, a decreased side effect and increased patient compliance.

A further object of the present invention is to obtain a combination composition having a desired level of compatibility.

Drugs of different action mechanisms can be combined. It is not possible, however, to state that a combination of drugs having different action mechanisms, but showing actions on similar targets, will have absolutely positive effects.

The term synergistic means that when drugs are administered together, a combined action is obtained which is higher than the individual actions of the respective drugs when they are used separately. On the other hand, using a lower dose of each drug to be combined according to the present invention will reduce the total dosage. Put differently, the dosages have not to be relatively less in all cases, but the drugs can be dosed less frequently or this may be beneficial in reducing the recurrence rate of side effects. These are advantageous in terms of patients to be treated.

The preferred dosages of active agents included to the pharmaceutical combination according to the present invention are therapeutically active dosages, and particularly correspond to the dosage of those which are commercially available. Therapeutically active amount not only includes therapeutic doses, but also preventive/prophylactic doses.

In one embodiment of this invention, the pharmaceutical compositions comprise the active agents in an amount ranging from 0.1% to 90%. The pharmaceutical composition comprise sofosbuvir in an amount of between 50 and 1500 mg and ribavirin in an amount of between 50 and 2000 mg.

Another embodiment of this invention, the therapeutically active amount of sofosbuvir and ribavirin is administrated once a day (QD) or twice a day (BID) and dosage regimen preferably is once a day (QD) for 6 weeks to 52 weeks.

The methods and formulations provided herein can be administered to any subject in need of therapy including, without limitation, humans or animals.

In one embodiment, these pharmaceutical combinations are administrated oral, parenteral, intranasal, sublingual, transdermal, transmucosal, ophthalmic, intravenous, pulmonary, intramuscular or rectal administration, and preferably oral administration.

The pharmaceutical composition of the invention is formulated preferably in the form of tablet or capsule or multilayer tablet.

The present invention relates to a pharmaceutical combination comprising immediate release phase of sofosbuvir and controlled release phase of ribavirin comprising a rate controlling agent that provides such therapeutic relief that dose dumping is prevented and requisite blood levels are maintained for an extended time period sufficient to justify once daily or twice daily dosing and thus increase patient compliance.

According to the embodiment of the present invention, sofosbuvir and/or ribavirin is in the form of controlled release, which, on average, and in a single dose per day in vitro release profile, the controlled release phase of the formulation does not dissolve more than 30% within 2 hours and does not dissolve more than 40-65% within 4 hours and is released by 85% at least in 12 hours.

In one embodiment, the pharmaceutical composition comprising an immediate release phase of sofosbuvir and a controlled release phase of ribavirin and at least one pharmaceutically acceptable excipient which is a rate controlling agent.

In one embodiment, the pharmaceutical composition comprising a controlled release phase of sofosbuvir and an immediate release phase of ribavirin and at least one pharmaceutically acceptable excipient which is a rate controlling agent.

In one embodiment, the pharmaceutical composition comprising a controlled release phase of sofosbuvir and a controlled release phase of ribavirin and at least one pharmaceutically acceptable excipient which is a rate controlling agent.

In one embodiment, the pharmaceutical composition of the invention is for use in the treatment of viral infections and preferably hepatitis C virus infections, chronic hepatitis C (CHC), hepatocellular carcinoma or patients with end-stage liver disease awaiting liver transplantation treating activity.

According to the challenges mentioned above the selection of the excipients thus very important. According to this embodiment, one or more pharmaceutically acceptable excipient is selected from the group comprising buffering agents, stabilizers, antioxidants, binders, diluents, dispersing agents, rate controlling agents, lubricants, glidants, disintegrants, plasticizers, preservatives, sweeteners, flavoring agents, coloring agents, optionally coating materials or mixtures thereof.

Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate, glycin , glutamic acid or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, ascorbic acid, gallic acid esters or the mixtures thereof, and preferably, citric acid, fumaric acid or mixtures thereof.

Suitable antioxidants may comprise but not limited to alpha tocopherol, ascorbic acid, ascorbyl palmitate, butylhydroxyanisole (BHA), butylhydroxytoluene (BHT), erythorbic acid, monothioglycerol, potassium metabisulfite, propyl gallate, sodium ascorbate, sodium metabisulfite, sodium sulfite, thymol or mixtures thereof.

Suitable binders may include but not limited to polyvinylpyrrolidone, polyethylene glycol, macrogol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable diluents may comprise but not limited to microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable dispersing agents may comprise but not limited to calcium silicate, magnesium aluminum silicate or mixtures thereof.

Suitable rate controlling agents may comprise but not limited to ethyl acrylate, ethyl methacrylate copolymer, ethylcellulose, methylcellulose, hypromellose phthalate, polydextrose, polyvinylacetate phthalate, zein, polyvinylpyrrolidone, polyvinyl alcohol, polyvinyl acetate, hydroxypropyl cellulose, hydroxypropyl methylcellulose, hydroxypropyl methylcellulose E4M, hydroxypropyl methylcellulose K100MCR, hydroxyethyl cellulose, hydroxymethyl cellulose, gelatin, polyethylene oxide, acacia, dextrin, starch, polyhydroxyethylmethacrylate, sodium carboxymethylcellulose, carboxymethyl cellulose, sodium alginate, alginic acid, pectin, polyglucoronic acid, polygalacturonic acid, chondroitic sulfate, carrageenan, lambda carregeenan, iota carregeenan, furcellaran, xanthan gum, a polymer of acrylic acid, carbopol, agar, gua gum, psyllium seed gum, gellan gum, locust bean gum, tara gum, tamarind gum, gum arabic, curdlan, galactomannan, glucomannan, nitrocellulose, methylcellulose, proteoglycan, glycoprotein, actin, tubulin, hemoglobin, insulin, fibrin, albumin, myosin, collagen, casein, pullulan, chitosan, glycerol, propylene glycol, macrogols, phfchalate esters, dibutyl sebacetate, citrate esters, triacetin, castor oil, acetylated monoglycerides, fractionated coconut oil, hydrogenated vegetable oil, hydrogenated castor oil, carnauba wax, candellia wax, beeswax, paraffin wax, stearic acid, glyceryl behenate, cetyl alcohol, cetostearyl alcohol, or a mixtures thereof.

According to the embodiment, rate controlling agent is selected from the group consisting of glyceryl behenate, xanthan gum, guar gum, polyethylene oxide, sodium alginate, ethyl cellulose, hydroxypropyl cellulose or hydroxypropyl methylcellulose E4M or hydroxypropyl methylcellulose K100MCR.

Suitable lubricants may comprise but not limited to magnesium stearate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable glidants may comprise but not limited to talc, aluminium silicate, colloidal silica, colloidal silicon dioxide, starch or mixtures thereof.

Suitable disintegrants may comprise but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof. Suitable plasticizers may comprise but not limited to polyethylene glycols of different molecular weights, propylene glycol or mixtures thereof.

Suitable preservatives may comprise but not limited to methyl paraben, propyl paraben and their salts (such as sodium, potassium), sodium benzoate, citric acid, benzoic acid, butylated hydroxytoluene or butylated hydroxyanisole, m-cresol, phenol or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol or mixtures thereof.

Suitable flavoring agents may comprise but not limited to menthol, peppermint, cinnamon, chocolate, vanillin, tutti frutti or fruit essences such as cherry, orange, strawberry, grape, black currant, raspberry, banana, red fruits, wild berries or mixtures thereof.

Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

In one embodiment of the invention, the pharmaceutical composition may comprise optionally a coating wherein the coating material is selected from the group comprising iron oxide yellow, polyvinyl alcohol-polyethylene glycol copolymers (Kollicoat IR), polyvinylalcohol based films (Opadry 200), polyvinyl alcohol or copolymers or mixtures thereof (Opadry AMB), hydroxypropyl methyl cellulose (HPMC), ethyl cellulose, ethylcellulose dispersions (Surelease), Kerry-HPC, polyethylene glycol, polyvinylprolidone, polyvinylprolidone-vinyl acetate copolymer (PVP-VA) and all kinds of OpadryTM, as well as pigments, dyes, titanium dioxide, iron oxide, talc, chromatone-P, triacetin or polymethylmetacrylate copolymers (Eudragit).

According to the pharmaceutical compositions of the invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is dried and sieved and optionally mixed with additional excipients. Finally, it is compressed into dosage forms. In addition, this novel pharmaceutical formulation is produced by various technologies such as tablet in tablet technology or fluidized bed granulation technique or extrusion/spheronization or spray drying or lyofilization.

### Examples:

### Example-1: Sofosbuvir IR phase + Ribavirin CR phase

| **Immediate release phase (Sofosbuvir)** | **% amount (w/w)** |
|---|---|
| Sofosbuvir | 5.00-95.00% |
| Lactose monohydrate | 5.00-75.00% |
| Corn starch | 5.00-30.00 % |
| Polyvinylpyrrolidone (PVP) | 0.50-5.00% |
| Microcrystalline cellulose (MCC) | 15.00-60.00% |
| Colloidal silicon dioxide | 0.10-1.00% |
| Magnesium stearate | 0.1-2.00% |

| **Controlled release phase (Ribavirin)** | **% amount (w/w)** |
|---|---|
| Ribavirin | 5.00-95.00% |
| Hydroxypropyl methylcellulose E4M (HPMC E4M) | 2.00-25.00% |
| Hydroxypropyl methylcellulose K100MCR (HPMC K100MCR) | 2.00-25.00% |
| Microcrystalline cellulose (MCC) | 15.00-60.00% |
| Colloidal silicon dioxide | 0.10-1.00% |
| Magnesium stearate | 0.10-2.00% |

### Immediate release phase (Sofosbuvir)

The process for the preparation of the immediate release phase of the pharmaceutical formulation, including the steps of:
a) Blending sofosbuvir, lactose monohydrate, corn starch, MCC;
b) granulating the blend of step a); with PVP;
c) sieved the blend of step b);
d) blending the granules of step c) with colloidal silicon dioxide and magnesium stearate.

### Controlled release phase (Ribavirin)

The process for the preparation of the controlled release phase of the pharmaceutical formulation, including the steps of:
a) Blending ribavirin, HPMC E4M and microcrystalline cellulose;
b) granulating the blend of step a); with water or without water (wet or dry granulation);
c) sieved the blend of step b);
d) blending the granules of step c) with HPMC K100MCR, colloidal silicon dioxide and mg stearate.

These different mixtures obtained are;
i. Pressed in the form of multilayer tablets. Optionally, this multilayer tablet can be coated.
ii. or filled into the capsules.

### Example-2: Sofosbuvir CR phase + Ribavirin IR phase

| **Controlled release phase (Sofosbuvir)** | **% amount (w/w)** |
|---|---|
| Sofosbuvir | 5.00-95.00% |
| Xanthan gum | 5.00-30.00% |
| Guar gum | 1.00-10.00% |
| Lactose monohydrate | 5.00-50.00% |
| Colloidal silicon dioxide | 0.10-2.00% |
| Magnesium stearate | 0.10-2.00% |

| **Immediate release phase (Ribavirin)** | **% amount (w/w)** |
|---|---|
| Ribavirin | 5.00-95.00% |
| Microcrystalline cellulose (MCC) | 5.00-50.00% |
| Corn starch | 5.00-30.00% |
| Crospovidone | 0.50-5.00% |
| Polyvinylpyrrolidone (PVP) | 0.50-5.00% |
| Talc | 0.50-5.00% |
| Magnesium stearate | 0.01-2.00% |

### Controlled release phase (Sofosbuvir)

The process for the preparation of the controlled release phase of the pharmaceutical formulation, including the steps of:
a) blending sofosbuvir, lactose monohydrate, xanthan gum and guar gum;
b) granulating the blend of step a); with water or without water (wet or dry granulation);
c) sieved the blend of step b);
d) blending the granules of step c) with colloidal silicon dioxide and magnesium stearate

### Immediate release phase (Ribavirin)

The process for the preparation of the immediate release phase of the pharmaceutical formulation, including the steps of:
a) Blending Ribavirin, MCC, corn starch, crospovidone;
b) granulating the blend of step a); with polyvinylpyrrolidone;
c) sieved the blend of step b);
d) blending the granules of step c) with talc and magnesium stearate.

These different mixtures obtained are;
i. Pressed in the form of multilayer tablets. Optionally, this multilayer tablet can be coated.
ii. or filled into the capsules.

### Example-3: Sofosbuvir CR phase + Ribavirin CR phase

| **Controlled release phase (Sofosbuvir)** | **% amount (w/w)** |
|---|---|
| Sofosbuvir | 5.00-95.00% |
| Polyethylene oxide | 0.50-25.00% |
| Sodium Alginate | 2.00-20.00% |
| Crospovidone | 1.00-5.00% |
| Polyethylene glycol | 1.00-10.00% |
| Aluminium silicate | 0.10-1.00% |
| Calcium stearate | 0.10-2.00% |

| **Controlled release phase (Ribavirin)** | **% amount (w/w)** |
|---|---|
| Ribavirin | 5.00-95.00% |
| Hydroxypropyl cellulose | 5.00-40.00% |
| Ethyl cellulose | 1.00-20.00% |
| Alginic acid | 1.00-5.00% |
| Stearic acid | 0.50-5.00% |
| Colloidal silicon dioxide | 0.10-2.00% |

### Controlled release phase (Sofosbuvir)

The process for the preparation of the controlled release phase of the pharmaceutical formulation, including the steps of:
a) blending sofosbuvir, polyethylene oxide and sodium alginate;
b) granulating the blend of step a); with polyethylene glycol;
c) sieved the blend of step b);
d) blending the granules of step c) with crospovidone, aluminium silicate and calcium stearate.

### Controlled release phase (Ribavirin)

The process for the preparation of the controlled release phase of the pharmaceutical formulation, including the steps of:
a) Blending Ribavirin, hydroxypropyl cellulose, ethyl cellulose;
b) granulating the blend of step a); with alginic acid;
c) sieved the blend of step b);
d) blending the granules of step c) with stearic acid and colloidal silicon dioxide.

These different mixtures obtained are;
i. Pressed in the form of multilayer tablets. Optionally, this multilayer tablet can be coated.
ii. Or filled into the capsules.

### Example 4: Sofosbuvir CR phase + Ribavirin IR phase (tablet in tablet technology)

| **Controlled release phase (Sofosbuvir)** | **% amount (w/w)** |
|---|---|
| Sofosbuvir | 5.00-95.00% |
| HPMC E4M | 2.00-25.00% |
| HPMC K100MCR | 2.00-25.00% |
| Lactose monohydrate | 5.00 - 50.00% |
| Colloidal silicon dioxide | 0.10-2.00% |
| Magnesium stearate | 0.10-2.00% |

| **Immediate release phase (Ribavirin)** | **% amount (w/w)** |
|---|---|
| Ribavirin | 5.00-95.00% |
| Microcrystalline cellulose (MCC) | 5.00-50.00% |
| Corn starch | 5.00-30.00% |
| Crospovidone | 0.50-5.00% |
| Polyvinylpyrrolidone (PVP) | 0.50-5.00% |
| Talc | 0.50-5.00% |
| Magnesium stearate | 0.01-2.00% |

### Controlled release phase (Sofosbuvir)

The process for the preparation of the controlled release phase of the pharmaceutical formulation, including the steps of:
a) blending sofosbuvir, lactose monohydrate and HPMC E4M;
b) granulating the blend of step a); with water or without water (wet or dry granulation);
c) sieved the blend of step b);
d) blending the granules of step c) with HPMC K100MCR, colloidal silicon dioxide and magnesium stearate

### Immediate release phase (Ribavirin)

The process for the preparation of the immediate release phase of the pharmaceutical formulation, including the steps of:
a) Blending Ribavirin, MCC, corn starch, crospovidone;
b) granulating the blend of step a); with polyvinylpyrrolidone (PVP);
c) sieved the blend of step b);
d) blending the granules of step c) with talc and magnesium stearate.

These different mixtures obtained are;
i. Used tablet in tablet technology. Optionally, this tablet can be coated.

## Claims

1. A pharmaceutical composition comprising sofosbuvir and ribavirin as active agents with at least one pharmaceutically acceptable excipient, wherein at least one of the active agents is in the form of controlled release.

2. The pharmaceutical composition according to claim 1, wherein sofosbuvir in an amount of between 50 and 1500 mg and ribavirin in an amount of between 50 and 2000 mg.

3. The pharmaceutical composition according to claim 1 or 2, wherein said composition is administrated once a day or twice a day and dosage regimen preferably is once a day for 6 weeks to 52 weeks.

4. The pharmaceutical composition according to claim 1 or 2, wherein said pharmaceutical composition is formulated preferably in the form of tablet or capsule or multilayer tablet.

5. The pharmaceutical composition according to claim 1, wherein said pharmaceutical composition comprising an immediate release phase of sofosbuvir and a controlled release phase of ribavirin and at least one pharmaceutically acceptable excipient which is a rate controlling agent.

6. The pharmaceutical composition according to claim 1, wherein said pharmaceutical composition comprising a controlled release phase of sofosbuvir and an immediate release phase of ribavirin and at least one pharmaceutically acceptable excipient which is a rate controlling agent.

7. The pharmaceutical composition according to claim 1, wherein said pharmaceutical composition comprising a controlled release phase of sofosbuvir and a controlled release phase of ribavirin and at least one pharmaceutically acceptable excipient which is a rate controlling agent.

8. The pharmaceutical composition according to claim 5 to 7, wherein the rate controlling agents are selected from the group comprising ethyl acrylate, ethyl methacrylate copolymer, ethylcellulose, methylcellulose, hypromellose phthalate, polydextrose, polyvinylacetate phthalate, zein, polyvinylpyrrolidone, polyvinyl alcohol, polyvinyl acetate, hydroxypropyl cellulose, hydroxypropyl methylcellulose, hydroxypropyl methylcellulose E4M, hydroxypropyl methylcellulose K100MCR, hydroxyethyl cellulose, hydroxymethyl cellulose, gelatin, polyethylene oxide, acacia, dextrin, starch, polyhydroxyethylmethacrylate, sodium carboxymethylcellulose, carboxymethyl cellulose, sodium alginate, alginic acid, pectin, polyglucoronic acid, polygalacturonic acid, chondroitic sulfate, carrageenan, lambda carregeenan, iota carregeenan, furcellaran, xanthan gum, a polymer of acrylic acid, carbopol, agar, gua gum, psyllium seed gum, gellan gum, locust bean gum, tara gum, tamarind gum, gum arabic, curdlan, galactomannan, glucomannan, nitrocellulose, methylcellulose, proteoglycan, glycoprotein, actin, tubulin, hemoglobin, insulin, fibrin, albumin, myosin, collagen, casein, pullulan, chitosan, glycerol, propylene glycol, macrogols, phfchalate esters, dibutyl sebacetate, citrate esters, triacetin, castor oil, acetylated monoglycerides, fractionated coconut oil, hydrogenated vegetable oil, hydrogenated castor oil, carnauba wax, candellia wax, beeswax, paraffin wax, stearic acid, glyceryl behenate, cetyl alcohol, cetostearyl alcohol, or a mixtures thereof.

9. The pharmaceutical combination according to to claim 8, wherein the rate controlling agent is selected from the group consisting of glyceryl behenate, xanthan gum, guar gum, polyethylene oxide, sodium alginate, ethyl cellulose, hydroxypropyl cellulose or hydroxypropyl methylcellulose E4M or hydroxypropyl methylcellulose K100MCR.

10. The pharmaceutical composition according to claim 1, wherein at least one pharmaceutically acceptable excipient other than rate controlling agent is selected from the group comprising buffering agents, stabilizers, antioxidants, binders, diluents, dispersing agents, lubricants, glidants, disintegrants, plasticizers, preservatives, sweeteners, flavoring agents, coloring agents, optionally coating materials or mixtures thereof.

11. The pharmaceutical composition according to any preceding claims comprising; immediate release phase of sofosbuvir;
a) 5.00 - 95.00 % by weight of sofosbuvir
b) 5.00 - 75.00 % by weight of lactose monohydrate
c) 5.00 - 30.00 % by weight of corn starch
d) 0.50 - 5.00 % by weight of polyvinylpyrrolidone
e) 15.00 - 60.00 % by weight of microcrystalline cellulose
f) 0.10 - 1.00 % by weight of colloidal silicon dioxide
g) 0.10 - 2.00 % by weight of magnesium stearate
and controlled release phase of ribavirin;
a) 5.00 - 95.00 % by weight of ribavirin
b) 2.00 - 25.00 % by weight of hydroxypropyl methylcellulose E4M
c) 10.00 - 17.50 % by weight of hydroxypropyl methylcellulose K100MCR
d) 15.00 - 60.00 % by weight of microcrystalline cellulose
e) 0.10 - 1.00 % by weight of colloidal silicon dioxide
f) 0.10 - 2.00 % by weight of magnesium stearate
and optionally film coating.

12. The pharmaceutical composition according to any preceding claims comprising; controlled release phase of sofosbuvir;
a) 5.00 - 95.00 % by weight of sofosbuvir
b) 5.00 - 30.00 % by weight of xanthan gum
c) 1.00 - 10.00 % by weight of guar gum
d) 5.00 - 50.00 % by weight of lactose monohydrate
e) 0.10 - 2.00 % by weight of colloidal silicon dioxide
f) 0.10 - 2.00 % by weight of magnesium stearate
and immediate release phase of ribavirin;
a) 5.00 - 95.00 % by weight of ribavirin
b) 5.00 - 50.00 % by weight of microcrystalline cellulose
c) 5.00 - 30.00 % by weight of corn starch
d) 0.50 - 5.00 % by weight of crospovidone
e) 0.50 - 5.00 % by weight of polyvinylpyrrolidone
f) 0.50 - 5.00 % by weight of talc
g) 0.01 - 2.00 % by weight of magnesium stearate
h) and optionally film coating.

13. The pharmaceutical composition according to any preceding claims comprising; controlled release phase of sofosbuvir;
a) 5.00 - 95.00 % by weight of sofosbuvir
b) 0.50 - 25.00 % by weight of polyethylene oxide
c) 2.00 - 20.00 % by weight of sodium alginate
d) 1.00 - 5.00 % by weight of crospovidone
e) 1.00 - 10.00 % by weight of polyethylene glycol
f) 0.10 - 1.00 % by weight of aluminium silicate
g) 0.10 - 2.00 % by weight of calcium stearate
and controlled release phase of ribavirin;
a) 5.00 - 95.00 % by weight of ribavirin
b) 5.00 - 40.00 % by weight of hydroxypropyl cellulose
c) 1.00 - 20.00 % by weight of ethyl cellulose
d) 1.00 - 5.00 % by weight of alginic acid
e) 0.50 - 5.00 % by weight of stearic acid
f) 0.10 - 2.00 % by weight of colloidal silicon dioxide
and optionally film coating.

14. The pharmaceutical composition according to any preceding claims comprising; controlled release phase of sofosbuvir;
a) 5.00 - 95.00 % by weight of sofosbuvir
b) 2.00 - 25.00 % by weight of hydroxypropyl methylcellulose E4M
c) 2.00 - 25.00 % by weight of hydroxypropyl methylcellulose K100MCR
d) 5.00 - 50.00 % by weight of lactose monohydrate
e) 0.10 - 2.00 % by weight of colloidal silicon dioxide
f) 0.10 - 2.00 % by weight of magnesium stearate
and immediate release phase of ribavirin;
a) 5.00 - 95.00 % by weight of ribavirin
b) 5.00 - 50.00 % by weight of microcrystalline cellulose
c) 5.00 - 30.00 % by weight of corn starch
d) 0.50 - 5.00 % by weight of crospovidone
e) 0.50 - 5.00 % by weight of polyvinylpyrrolidone
f) 0.50 - 5.00 % by weight of talc
g) 0.01 - 2.00 % by weight of magnesium stearate
and optionally film coating.

15. The pharmaceutical composition according to any preceeding claims, for use in the treatment of viral infections and preferably hepatitis C virus infections, chronic hepatitis C, hepatocellular carcinoma or patients with end-stage liver disease awaiting liver transplantation treating activity.
